# NEUE EUROPÄISCHE PATENTSCHRIFT

(11) **EP 1 541 126 B2**
(45) Veröffentlichungstag und Bekanntmachung des Hinweises auf die Entscheidung über den Einspruch: **05.08.2015**
(45) Hinweis auf die Patenterteilung: 16.07.2008
(21) Anmeldenummer: 04105637.5
(22) Anmeldetag: 09.11.2004
(51) Int. Cl.: A61K 8/97, A61P 17/02, A61K 8/42, A61Q 19/00, A61Q 17/04

(54) **Kosmetische oder dermatologische Zubereitungen enthaltend eine Kombination aus grünem Farbstoff und antiinflammatorischem Wirkstoff**
Cosmetic and dermatological composition comprising a combination of a green colouring agent and an antiinflammatory agent
Composition cosmétique ou dermatologique comprenant la combinaison d'un agent colorant vert et d'un agent anti-inflammatoire

(30) Priorität: 04.12.2003 DE 10357046
(43) Veröffentlichungstag der Anmeldung: 15.06.2005
(73) Patentinhaber: Beiersdorf AG, 20253 Hamburg (DE)
(72) Erfinder: Kröpke, Rainer, 22869, Schenefeld (DE); Bürger, Anette, 22303, Hamburg (DE); Kolbe, Ludger, 21255, Dohren (DE); Mundt, Claudia, 28207, Bremen (DE)

(56) Entgegenhaltungen:
- EP-A1- 1 216 693
- EP-B1- 1 156 772
- WO-A-02/00190
- WO-A-02/15873
- WO-A-03/105787
- WO-A1-96/03962
- WO-A2-01/52795
- GB-A- 1 529 599
- US-A1- 2003 091 666

## Beschreibung

Die vorliegende Erfindung betrifft kosmetische und/oder dermatologische Zubereitungen enthaltend eine Kombination aus grünem Farbstoff und antiinflammatorischen Wirkstoff, insbesondere Zubereitungen zur Prophylaxe und Behandlung von sonnengereizter Haut und zur Unterstützung der körpereigenen Reparaturmechanismen.

Ferner betrifft die Erfindung die Verwendung solcher Zubereitungen, solche Kombinationen enthaltend.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

"Stinging"-Phänomene können als kosmetisch zu behandelnde Störungen angesehen werden. Starker Juckreiz dagegen, insbesondere bei Atopie auftretendes starkes Hautjucken sowie Juckreiz bei Hauterkrankungen, kann auch als schwerwiegendere dermatologische Störung bzw. neurosensorisches Phänomen bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen - z. B. Massage, Einwirkung (waschaktiver) Tenside, Wettereinfluß wie Sonne, Kälte, Trockenheit, aber auch feuchte Wärme, Wärmestrahlung und UV-Strahlung, z. B. der Sonne - hervorgerufen werden.

In "Journal of the Society of Cosmetic Chemists" 28, S.197 - 209 (Mai 1977) beschreiben P. J. Frosch und A. M. Kligman eine Methode zur Abschätzung des "Stinging-Potentials" topisch verabreichter Substanzen. Als positive Substanzen werden hier z. B. Milchsäure und Brenztraubensäure eingesetzt. Bei Messung nach dieser Methode wurden aber auch Aminosäuren, insbesondere Glycin, als neurosensorisch aktiv ermittelt (solche Substanzen werden "Stinger" genannt).

Nach bisherigen Erkenntnissen tritt eine derartige Empfindlichkeit gegenüber ganz bestimmten Substanzen individuell unterschiedlich auf. Dies bedeutet, daß eine Person, die bei Kontakt mit einer Substanz "Stingingeffekte" erlebt, sie mit hoher Wahrscheinlichkeit bei jedem weiteren Kontakt wiederholt erleben wird. Der Kontakt mit anderen "Stingern" kann aber ebensogut ohne jede Reaktion verlaufen.

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten ferner auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Auch die Rasur induziert in dafür empfindlichen Personen Erytheme, Brennen, Juckreiz und Spannungsgefühle, die durch die oberflächliche Verletzung und die mechanische Belastung der obersten Hautschichten sowohl bei der Nassrasur als auch bei der Trockenrasur ausgelöst werden. Diese Beschwerden treten häufig bei der täglichen Rasur der Barthaare auf, aber auch nach der Rasur von Achsel-, Scham-, und Beinbehaarung können Irritationen auftreten.

Neben den positiven Auswirkungen des Sonnenlichtes, wie dem allgemeinen Wohlbefinden, der Bildung von Vitamin D3 und der Aknebehandlung, gibt es auch negative Auswirkungen, denen es entgegenzuwirken gilt.

Die Bedingungen eines Sonnenbades stellen für den menschlichen Organismus eine ungewohnte, zum Teil extreme Belastung dar, von der insbesondere die Haut betroffen ist. Solange die Strahlenbelastung ein gewisses Ausmaß nicht überschreitet, wird unsere Haut damit fertig. Geringere Schäden, wie sie bei nicht spürbaren Suberythemen vorliegen, werden sofort behoben.
Setzt man die Haut allerdings zu lange der Sonne oder einer künstlichen Strahlenquelle aus, so entwickelt sich nach einer Latenzzeit von 2 bis 3 Stunden eine gegen die unbestrahlte Haut stark abgegrenzte Hautrötung, das Erythema solare. Bei dem so entstehenden Sonnenbrand unterscheidet man zwischen
■ 1. Grad: Erythem (Rötung, Wärmegefühl, Brennen, Spannungsgefühl der Haut) klingt nach 2 bis 3 Tagen wieder ab und verschwindet unter gleichzeitig zunehmender Pigmentierung,
■ 2. Grad: Blasenbildung
   auf der Haut bilden sich Blasen mit Brennen und Jucken, die Oberhaut wird flächig abgestoßen
■ 3. Grad: Zellschädigung
   es treten tiefgehende Zellschädigungen auf, der Körper reagiert mit Fieber, die Oberhaut wird großflächig abgestoßen.
Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.
Sonnenhungrige Urlauber setzen sich gerade zu beginn des Urlaubs sehr stark der Sonne aus. Dadurch ist die Gefahr von gereizter, rötlicher Haut oder sogar ein lewichter Sonnenbrand sehr groß.
Der Sonnenbrand bzw. das Lichterythem sind die akuten Erscheinungsformen der Lichteinwirkung. Neben den bekannten schädigenden Wirkungen der UV-Strahlen kommt es in der Nachreaktion der Haut ferner zu einer verminderten Sebumproduktion und einem Austrocknen der Haut. Zur Behandlung der Haut dienen die sogenannten Aftersun-Präparate, deren Anwendung grundsätzlich nach jeder Sonnenexposition empfohlen wird. Dabei handelt es sich in der Regel um Emulsionen oder wäßrige Hydrogele, die neben üblichen Feuchthaltesubstanzen auch spezielle Wirkstoffe enthalten können, wie beispielsweise
■ entzündungslindernde und kühlende Stoffe,
■ lokal anaestesierende Stoffe und/oder
■ desinfizierende Stoffe, um mögliche Hautinfektionen zu verhindern.

Eingesetzt werden z. B. aus Pflanzen gewonnene entzündungslindernde bzw. -hemmende Wirkstoffe wie Azulen und Bisabolol (Kamille), Glycyrrhizin (Süßholzwurzel), Hamamelin (Hamamelis) oder Gesamtextrakte, z. B. aus Aloe vera oder Kamille. Diese zeigen bei leichteren Formen und lokal begrenzten Erythemreaktionen gewisse Erfolge. Gleiches gilt für Cremes mit einem hohen Gehalt an ätherischen Ölen oder Panthenol.

Aftersun-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird beispielsweise durch hohe Mengen an Ethanol erzielt, welches beim Verteilen der Formulierung auf der Haut spontan verdunstet. Auch Hydrogele, O/W-Emulsionen (Lotionen) oder wäßrige Schüttelmixturen haben durch die Verdunstungskälte der wäßrigen Phase einen ausgeprägten Kühleffekt, der über eine lokale Gefäßverengung zu einer Entzündungsmilderung führt.

Neben diesem durch Sonnenbestrahlung verursachten unansehnlichen rötlichen Erscheinungsbild der Haut leiden vielen Menschen an Couperosis (Gefäßerweiterungen im Gesicht), Rosacea oder anderen ähnlichen Krankheitsbildern, die mit einer unnatürlich geröteten Haut einhergehen.

Rosacea ist eine erblich bedingte, nicht ansteckende Hauterkrankung, die in der Regel nicht vor dem 30. Lebensjahr ausbricht; die Betroffenen befinden sich häufig bereits im 5. Lebensjahrzehnt. Dann kommt es zu einer Erweiterung der Blutgefäße, welche die Haut rot "aufblühen" lässt. Phasenweise können auch Entzündungen um die Talgdrüsen auftreten. Diese entzündlichen Vorgänge verursachen Eiterbläschen und Pusteln, haben jedoch nichts mit Akne zu tun.

Die Hautkrankheit Rosacea bedeutet übersetzt soviel wie "Rosenblütchen". Dies spielt auf die Rötung im Gesicht an, die für Rosacea typisch ist. Neben diesen Rötungen, die durch erweiterte Blutäderchen entstehen, kann es durch Entzündungen auch zu Veränderungen der Nase kommen.

Zwar ist bis heute die Ursache von Rosacea nicht eindeutig geklärt, die Grundlage ist jedoch offenbar die sogenannte Rosacea-Diathese. Das heißt, die Neigung, auf bestimmte Reize mit ausgeprägten Gesichtsrötungen zu reagieren, die nach einer Weile wieder abklingen. Dieser Rötungszustand wird auch Flush genannt.
Die Rötung des Gesichts als Reaktion auf äußere Faktoren (Temperaturwechsel, scharfe Speisen) kann jederzeit auftreten und klingt dann normalerweise wieder ab. Bei Menschen mit einer Rosacea-Neigung kann diese verstärkte Durchblutung zu bleibend erweiterten Äderchen führen (sogenannte Teleangiektasien), die als fadenfeine, rote Linien auf der Haut sichtbar sind. Diese erweiterten Äderchen kommen insbesondere rund um die Talgdrüsen des Gesichts vor und sind das erste Anzeichen einer Rosacea.

WO 0200190 beschreibt kosmetische oder pharmazeutische Zubereitungen, die einen oder mehrer Inhaltstoffe beinhalten, die grün phosphoreszierendes Licht emittieren. Grünes Licht hat bekanntermaßen einen antioxidativen Effekt (Comorosan Effekt).

US 2003091666 A1 beschreibt topische Zusammenstzungen zur Behandlung entzündlicher Hautkrankheiten wie z.B. Rosacea enthaltend 0,5 - 10 Gew.% eines Extraktes von Morinda citrifolia. In den Zubereitungen können u.a. zusätzich Grünpigmente enthalten sein.

WO 0215873 beschreibt topische Zubereitungen bei denen entzündungshemmende Wirkstoffe zur behandlung entzündlicher Hautsymptome eingestzt werden.

Aus der dekorativen Kosmetik sind darüber hinaus Zubereitungen bekannt, die aufgetragen auf die Haut, diese Hautareale farbig abdecken.

Aufgabe der vorliegenden Erfindung war es, den Nachteilen des Standes der Technik abzuhelfen und Zubereitungen zur Verfügung zu stellen, die der geschädigten Haut ein natürliches Aussehen zurückgeben. Insbesondere war die Aufgabe der vorliegenden Erfindung Zubereitungen zur Verfügung zu stellen, die auf durch Sonnenbestrahlung oder krankheitsbedingten Umständen, wie Rosacea, geröteter Haut aufgetragen werden können und zu einer Prophylaxe und Behandlung von sonnengereizter Haut und zur Unterstützung der körpereigenen Reparaturmechanismen führen.

Gelöst werden die angeführten Aufgaben durch Zubereitungen gemäß dem Hauptanspruch. Gegenstand der Unteransprüche sind vorteilhafte Ausführungsformen der erfindungsgemäßen Zubereitungen. Des weiteren umfasst die Erfindung die Verwendung derartiger Zubereitungen.

Es war überraschend und darin liegt die Lösung dieser Aufgaben, dass kosmetische oder dermatologische Zubereitungen mit einem Gehalt an 0,01Gew.% - 5 Gew.% rotlichtfilternder Farbstoffe, 0,0001 Gew.% - 10 Gew.% antiinflammatorisch (antiendzündlich wirkende) Wirkstoffe, Hammamelis, Glycyrhizza inflata, Panthenol und/oder Kamillenextrakt oder Licochalcon A ein oder mehrere Weißpigmente und ein oder mehrere Blaupigmente im Verhältnis 1:1 bis 1:100 im Bezug auf das eingesetzte Grünpigment, den Nachteilen des Standes der Technik abhelfen. Die Prozentangaben beziehen sich auf die Gesamtmasse der Zubereitungen.

Die erfindungsgemäßen Formulierungen sind in jeglicher Hinsicht überaus befriedigende Präparate, die sich durch eine hervorragende Wirkung auszeichnen. Bei Anwendung der erfindungsgemäß verwendeten Wirkstoffe bzw. kosmetischer oder topischer dermatologischer Zubereitungen mit einem wirksamen Gehalt an erfindungsgemäß verwendeten Wirkstoffen ist eine wirksame Behandlung, aber auch eine Prophylaxe von entzündlichen Hautzuständen, Rosacea und insbesondere sonnengereizter Haut möglich.

Die erfindungsgemäße Zubereitungen ermöglichen einerseits eine visuelle Kaschierung von Rosacea und geröteter Haut, insbesondere durch Sonnebestrahlung verursacht, und andererseits eine langanhaltende Pflege der Haut.
Es ergibt sich eine überraschend synergistische Wirkung aufgrund der Kombination der erfindungsgemäßen Bestandteile:
ein Soforteffekt durch die rotlichtfilternden Farbstoffe (Grünpigmente) und ein Langzeiteffekt durch die antiendzündlichen Wirkstoffe.

Es war für den Fachmann nicht vorauszusehen gewesen, dass die erfindungsgemäßen Zubereitungen
■ lichtstrapazierte Haut besser pflegen,
■ die Nachreaktionen der Haut auf die Einwirkung von UV-Strahlung besser vermindern,
■ die vom Sonnenbaden gereizte Haut besser beruhigen,
■ leichten Sonnenbrand schneller zum Abklingen bringen würden,
■ gerötete Hautareale normal aussehend erscheinen läßt,
■ natürliches Aussehen zurückbringt,
■ einfacher zu formulieren ist,
■ sich durch besser Pflegewirkung auszeichen würden
als die Zubereitungen des Standes der Technik.

Die Erfindung ist selbstverständlich nicht auf Zubereitungen beschränkt, welche nach dem Sonnenbad angewendet werden, sondern umfaßt naturgemäß alle kosmetischen und dermatologischen Anwendungen, bei welchen eine entzündungslindernde Wirkung gewünscht oder von Vorteil sein könnte. Hier sei insbesondere auch der Rasurbrand genannt, wie er nach der Rasur häufig auftritt.

Gegenstand der Erfindung ist daher ferner die Verwendung kosmetischer oder dermatologischer Formulierungen zur Pflege licht- und rasurstrapazierter Haut und/oder zur Linderung oder der Nachreaktionen der Haut auf die Einwirkung von UV-Strahlung und/oder der Rasur sowie zur Behandlung und Pflege krankheitsbedingt rotgefärbuter Haut, wie beispielsweise Rosacea.

Als antiinflammatorische Wirkstoffe gelten Hammamelis, Glycyrhizza inflata, Panthenol und Kamillenextrakt sowie Kombinationen daraus.

Die Pflanzenart *Glycyrrhiza inflata* gehört wie das in Europa offizielle Süßholz *Glycyrrhiza glabra* der Gattung *Glycyrrhiza* an, die zur Pflanzenfamilie der *Fabaceae* (Erbsengewächse) gehört. Die Droge *Radix Glycyrrhizae inflatae*, d.h., die Wurzel der Pflanze, ist, beispielsweise in der fernöstlichen Medizin, gebräuchlich. Die Verwendung der Droge als Entzündungshemmer ist ebenfalls bekannt.

Ein Bestandteil des wäßrigen Auszugs aus Radix Glycyrrhizae inflatae ist das Licochalcon A, welches sich durch die folgende Strukturformel auszeichnet:

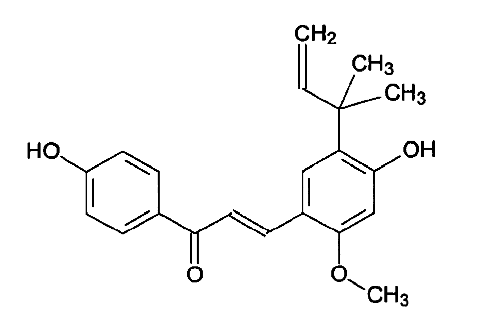

Es wird angenommen, daß diese Substanz, möglicherweise in Synergie mit den übrigen Bestandteilen des Extraktes, Anteil an der erfindungsgemäßen Wirkung besitzt.
Die antiinflammatorische Wirkung von Licochalcon A ist an sich bekannt. Zur Erzielung dieser Wirkung in topischen Formulierungen ist jedoch die Löslichkeit von Licochalcon A im Vehikel und eine ausreichende dermale Bioverfügbarkeit eine wichtige Voraussetzung. Licochalcon A ist jedoch eine schwer lösliche Substanz, die während der Lagerung und Verarbeitung auskristallisieren kann. Diese Kristallisationsneigung stellt ein großes Problem dar, da Wirkstoffkristalle zu unkosmetischer Anmutung, Formelinstabilitäten und/oder Wirkverlust führen können. Es ist daher erfindungsgemäß durch die erfindunsgemäßen Zubereitungen gelöst worden, die Löslichkeit von Licochalcon A und damit dessen biologische Verfügbarkeit zu erhöhen.
Erfindungsgemäß sind demnach insbesondere auch kosmetische oder dermatologische Zubereitungen mit einem Gehalt an Licochalcon A in Kombination mit rotlichtfilternden Farbstoffen.

Der Synergismus zeigt sich überraschenderweise besonders vorteilhaft, wenn die kosmetischen oder dermatologischen Zubereitungen 0,01 Gew.% bis 10 Gew.-%, insbesondere 0,05 bis 5 Gew.-%, ganz besonders 0,1 bis 2 Gew.-% an antiinflammatorischen Wirkstoff, insbesondere einem wäßrigen Extrakt aus *Radix Glycyrrhizae inflatae*, enthalten.

Ganz besonders vorteilhaft ist es, von einem wäßrigen Extrakt auszugehen, der unter der Bezeichnung Aqua Licorice Extract P-U der Firma Maruzen bekannt ist, der ein wäßriges Gemisch (ca. 10 Gew.-% Wasser) aus *Radix Glycyrrhizae inflatae* (ca. 5 Gew.%, Anteil Licochalcone A im Extrakt ca. 22 %, PPG-6 Decyltetradeceth-30 (ca. 25 Gew.-%) und Butylenglycol (ca. 60 Gew.-%) darstellt. Der Extrakt kann dabei vorteilhaft zu einem Anteil 0,01 bis 10 Gew.% zugesetzt werden.

Ferner ist es vorteilhaft, Licochalcone A in anderen Vehikelsystemen in einer Konzentration von 0,0001 bis 5 Gew.-%, insbesondere 0,001 bis 1 Gew.-%, ganz besonders 0,005 - 0,25 Gew.-% zu verwenden. Der Wirksstoffanteil an Licochalcone ist bezogen auf die Gesamtmasse der Zubereitung.

Hamamelis ist ein weiterer bekannter antiinflammatorisch wirkender Stoff. Hamamelis, in der nordamerikanischen Heimat als Zaubernuß bekannt, ähnelt unserem Haselnußstrauch und wird deshalb teilweise auch Hexenhasel genannt. Die Hamamelisblätter enthalten ätherische Öle, Gerbstoffe (Gallotannien) und Flavonoide, die die Pflanze zur Heilpflanze machen. Hamamelis wirkt entzündungshemmend, antiseptisch, zusammenziehend, abschwellend, lokal blutstillend, blutdrucksenkend, gefäßwandschützend. Hamamelis findet Anwendung in vielen kosmetischen Pflegepräparaten zur Hautpflege, in Gesichts-, Rasier- und Haarwässern, bei schlecht heilenden Wunden, Hautentzündungen, Hautrötungen, Muskelkater, Hexenschuß, Krampfadern, Hämorrhoiden, Venenerkrankungen wie Varizen, rheumatischen Beschwerden und Haarproblemen.

Panthenol, freie internat. Kurzbezeichnung für (±)-2,4-Dihydroxy-*N*-(3-hydroxypropyl)-3,3-dimethylbutyramid (Dexpanthenol, CTFA-Bez.: Panthenol). 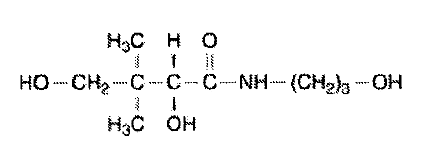 C₉H₁₉NO₄, M_{R} 205,25. Lediglich die D-Form wird als Vitamin, das im Organismus in Pantothensäure umgewandelt wird, verwendet. Die Pantothensäure soll das Haarwachstum begünstigen, weshalb es zur Herstellung von Haarwässern verwendet wird. Des weiteren wirkt Panthenol lindernd bei Sonnenbrand und stimuliert die Pigmentation. Panthenol ist häufig Bestandteil von Feuchtigkeitscremes und zeigt einen Moisturizing-Effekt. Panthenol wird vorwiegend zur Behandlung von Entzündungen des Magen-Darm-Kanals, des Auges u. der Haut, auch bei Wundheilungsstörungen sowie in Haarwässern u. a. Kosmetika eingesetzt.

Rotlichtfilternde Farbstoffe, insbesondere Grünpigmente, können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch.
Vorteilhafte Grünpigmente können aus der folgenden Gruppe allein oder in Kombination ausgewählt werden: Colorona Brilliant Green, Colorona Egyptian Emerald, Timiron Splendid Green, Vert Oxyde Crome Anhydre N, Vert Oxydede Crome Hydrate W886 und/oder Outremer Supercosmetique W 6803. Diese genannten Pigemente werden u.a. auch unter den entsprechenden Cl Nummern gelistet. Bevorzugt sind daher insbesondere die Grünpigmente mit den Cl Nummern Cl 77288, Cl 77289.
Entscheidend für den Farbstoff ist, dass er auf rötlichem oder rotem Hintergrund aufgebracht ein hautfarbendes Gesamterscheinungsbild vermittelt. Erfindungsgemäß werden daher 0,01Gew.% bis 5 Gew.%, bevorzugt 0,08 bis 0,25 Gew.%, insbesondere 0,1 bis 0,2 Gew.%, bezogen auf die Gesamtmasse der Zubereitung, rotlichtfilternde Farbstoffe eingesetzt.

Rotlichtfilternd bedeutet hierin, dass der Rotlichtanteil der sichtbaren Lichtes absorbiert wird und die Komplementärfarbe Grün sichtbar wird.

Durch den Auftrag der erfindungsgemäßen Zubereitung mit rotlichtfilternden Farbstoffen, wie beispielsweise die angegebenen Grünpigmente, auf die Haut, die beispielsweise durch Sonnenbestrahlung oder aufgrund Rosacea rötlich gefärbt erscheint, wird für den Betrachter das Erscheinungsbild der Haut natürlich erscheinen. Um diesen Effekt zu generieren ist die Auswahl und Menge an rotlichtfilternden Farbstoffen wesentlich.
Darüber hinaus ist auch ein Zusatz von Weisspigment erfindungsgemäß, insbesondere alle deckenden, weißen Pigmente, insbesondere alle Titandioxide, Wismutoxide, Bariumsulfat. Die Weißpigmente decken die gerötete Haut leicht ab und geben eine gewisse Deckkraft. Ohne Weisspigmente benötigt somit eine höheren Anteil an den anderen Farbpigmenten. Ebenso ist der Zusatz von Blaupigmenten im Verhältnis 1:1 bis 1:100 im Bezug auf das eingesetzte Grünpigment erfindungsgemäß. Das Blaupigment erhöht die farbverändernde Wirkung, so dass die rötliche Hautfarbe in ein natürliches Braun, d.h. eine natürliche Hautfarbe, besser umgewandelt wird. Erfindungsgemäß ist diese spezielle Farbwirkung mit Weiß- bzw. Blaupigmenten durch den Einsatz der rotlichtfilternden Farbstoffe in einem Anteilsbereich von 0,01 bis 5 Gew.% möglich.

Es ist bevorzugt im Sinne der vorliegenden Erfindung, wenn die erfindungsgemäßen kosmetischen oder dermatologischen Zubereitungen einen oder mehrere Alkohole enthalten, insbesondere, wenn die Formulierungen in Form eines Aftersun-Präparats vorliegen und sich durch eine besondere Kühlwirkung auszeichnen sollen.

Die kosmetischen oder dermatologischen Formulierungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Emulsionen können vorzugsweise auch eine Mikroemulsion, eine Pickering-Emulsion oder eine sprühbare Emulsion sein.

Vorzugsweise enthalten die erfindungsgemäßen Formulierungen ferner weitere entzündungshemmende Substanzen, wie z. B. Allantoin, α-Bisabolol, Panthotensäure, Gelée Royal, Azulen oder Aloe-vera-Extrakt sowie unverseifbare Anteile des Avocado- oder Sojaöls und weitere Substanzen, die die gereizte Haut beruhigen. Weitere vorteilhafte Wirkstoffe sind Gerbstoffe, welche adstringierende, entzündungshemmende und/oder sekretionshemmende Wirkung haben.

Darüberhinaus können die erfindungsgemäßen Formulierungen auch vorteilhaft Dihydroxyaceton oder Nußextrakte enthalten sowie weitere Substanzen, welche die Bräune erhalten sollen.

Die erfindungsgemäßen kosmetischen und/oder dermatologischen Formulierungen können wie üblich zusammengesetzt sein und insbesondere zur Behandlung und der Pflege der Haut und/oder der Haare nach einem Sonnenbad und als Schminkprodukt in der dekorativen Kosmetik dienen. Entsprechend können die erfindungsgemäßen Formulierungen - je nach ihrem Aufbau - beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Formulierungen als Grundlage für pharmazeutische Formulierungen zu verwenden. Bevorzugt sind insbesondere solche kosmetischen und dermatologischen Formulierungen, die in der Form eines Aftersun-Hautpflegeproduktes oder eines After-shave-Produktes vorliegen.

Zur Anwendung werden die erfindungsgemäßen kosmetischen und dermatologischen Formulierungen in der für Kosmetika üblichen Weise, d. h. beispielsweise direkt - nach der Entnahme aus einer Flasche, Tube, einem Tiegel oder einem anderen Behältnis - oder mit Hilfe eines (getränkten) Tuches auf die Haut und/oder die Haare in ausreichender Menge aufgebracht.

Getränkte Tücher finden als Gegenstände des täglichen Bedarfs breiten Einsatz in unterschiedlichsten Bereichen. Sie erlauben unter anderem effiziente und hautschonende Reinigung und Pflege besonders auch in der Abwesenheit von (fließendem) Wasser.
Dabei besteht der eigentliche Gebrauchsgegenstand aus zwei Komponenten:
a) einem trockenen Tuch, welches aus Materialien wie Papier und/oder unterschiedlichsten Mischungen aus Natur- oder Kunstfasern aufgebaut ist und
b) einer niederviskosen Tränkungslösung.

Gegenstand der vorliegenden Erfindung sind daher auch kosmetische und dermatologische Tücher, welche mit kosmetischen oder dermatologischen Tränkungslösungen befeuchtet sind, die einen Gehalt an erfindungsgemäßer Wirkstoffkombination aufweisen.

Erfindungsgemäß bevorzugte "trockene" Tücher (gemäß a)) bestehen aus Vlies, insbesondere aus wasserstrahlverfestigten und/oder wasserstrahlgeprägten Vlies.

Derartige Vliese können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Die Dicke des Vlieses beträgt vorzugsweise 0,4 mm bis 2 mm, insbesondere 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 % Viskose und 30 % PET.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Fernen weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Es ist vorteilhaft im Sinne der vorliegenden Erfindung, wenn das Gewichtsverhältnis des ungetränkten Tuchs zu der Tränkungslösung aus dem Bereich von 2 : 1 bis 1 : 6 gewählt wird.

Die im Rahmen der Beschreibung der vorliegenden Erfindung genannten erfindungsgemäßen kosmetischen und dermatologischen Formulierungen bzw. Zubereitungen stellen vorteilhafte Tränklösungen für kosmetische und dermatologische Tücher im Sinne der vorliegenden Erfindung dar.

Es ist vorteilhaft, wenn die erfindungsgemäßen Tränklösungen dünnflüssig, insbesondere sprühbar sind und z. B. eine Viskosität von weniger als 2000 mPa·s, insbesondere weniger als 1.500 mPa·s haben (Meßgerät: Haake Viskotester VT-02 bei 25 °C).

Bevorzugte Anwendungsform der erfindungsgemäßen Zubereitung ist daher eine Emulsion, ein Aerosol, ein Concealer (Abdeckstift) oder ein Gel.
Eine erfindungsgemäße Zubereitung als Concealer (Abdeckstift) ist besonders geeignet und damit bevorzugt, da auch kleinste Hautareale gezielt behandelt werden können. In bekannte Rahmenrezepturen für geeignete Abdeckstifte, die dem Fachmann geläufig sind, werden erfindungsgemäß die synergistische Kombination aus Farbstoff und Wirkstoff eingefügt und somit erfindungsgemäße Zubereitungen erhalten.

Die erfindungsgemäßen kosmetischen und dermatologischen Formulierungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel und/oder Silikonderivate sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch trans epidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Glycerin kann als Moisturizer im Sinne der vorliegenden Anmeldung im Bereich von 0,05-30 Gew.%, besonders bevorzugt sind 1-10%, eingesetzt werden.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Melanine, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Alanindiessigsäure, Flavonoide, Polyphenole, Catechine, Vitamin C und Derivate (z. B. Ascorbylpalmitat, Mg-Ascorbylphosphat, Ascorbylacetat), Tocopherole und Derivate (z. B. Vitamin-Eacetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Handelsname** | **INCI-Name** | **Polarität [mN/m]** |
|---|---|---|
| Isofol 14 T | Butyl Decanol (+) Hexyl Octanol (+) | 19,8 |
| | Hexyl Decanol (+) Butyl Octanol | |
| Lipovol MOS-130 | Tridecyl Stearate(+) Tridecyl Trimellitate(+) | 19,4 |
| | Dipentaerythrityl | |
| | Hexacaprylate/Hexacaprate | |
| Ricinusoel | | 19,2 |
| Isofol Ester 0604 | | 19,1 |
| Miglyol 840 | Propylene Glycol Dicaprylate/Dicaprate | 18,7 |
| Isofol 12 | Butyl Octanol | 17,4 |
| Tegosoft SH | Stearyl Heptanoate | 17,8 |
| Avocadocel | | 14,5 |
| Cetiol B | Dibutyl Adipate | 14,3 |
| Dermol 488 | PEG 2 Diethylenhexanoate | 10,1 |
| Cosmacol ELI | C12-13 Alkyl Lactate | 8,8 |
| Dermol 489 | Diethylen Glycol Dioctanoate(/ | 8,6 |
| | Diisononanoate | |
| Cosmacol ETI | Di-C12/13 Alkyl Tartrate | 7,1 |
| Emerest 2384 | Propylene Glycol Monoisostearate | 6,2 |
| Myritol 331 | Cocoglycerides | 5,1 |
| Prisorine 2041 GTIS | Triisostearin | 2,4 |

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 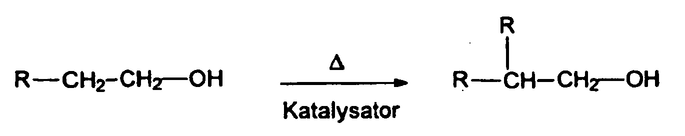 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 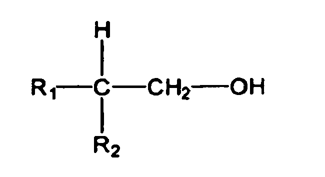 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 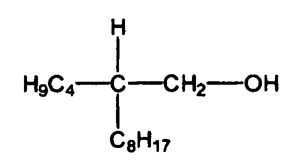 und ist beispielsweise unter der Handelsbezeichnung Isofol^{®} 12 von der Gesellschaft Condea Chemie GmbH erhältlich - und das 2-Hexyldecanol - es hat die chemische Struktur 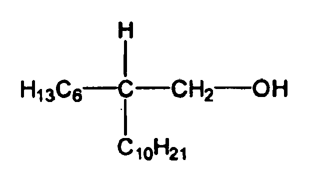 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 - 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Lipide im Sinne der vorliegenden Erfindung sind die im folgenden aufgelisteten Substanzen:

| **Handelsname** | **INCI-Name** | **Polarität [mN/m]** |
|---|---|---|
| DUB VCl 10 | Isodecyl Neopentanoate | 29,9 |
| Dermol IHD | Isohexyldecanoate | 29,7 |
| Dermol 108 | Isodecyl Octanoate | 29,6 |
| Dihexyl Ether | Dihexyl Ether | 29,2 |
| Dermol 109 | Isodecyl 3,5,5 Trimethyl Hexanoate | 29,1 |
| Cetiol SN | Cetearyl Isonananoate | 28,6 |
| Isopropylpalmitat | Isopropylpalmitat | 28,8 |
| DC Fluid 345 | Cyclomethicone | 28,5 |
| Dow Corning Fluid 244 | Cyclopolydimethylsiloxan | 28,5 |
| Jojobaöl Gold | | 26,2 |
| Wacker AK 100 | Dimethicone | 26,9 |
| Dermol 98 | 2- Ethylhexanosäure 3,5,5 Trimethylester | 26,2 |
| Dow Corning Fluid 246 | Offen | 25,3 |
| Eutanol G | Octyldodecanol | 24,8 |
| Isofol 16 | Hexyl Decanol | 24,3 |
| Dermol 139 | Isotridecyl 3,5,5 Trimethylhexanonanoate | 24,5 |
| Cetiol PGL | Hexyldecanol (+) Hexyl Decyl Laurate | 24,3 |
| Cegesoft C24 | Octyl Palmitate | 23,1 |
| M.O.D. | Octyldodeceyl Myristate | 22,1 |
| Macadamia Nut Oil | | 22,1 |
| Silikonöl VP 1120 | Phenyl Trimethicone | 22,7 |
| Isocarb 12 | Butyl Octanoicacid | 22,1 |
| Isopropylstearat | Isopropyl Stearate | 21,9 |
| Finsolv TN | C12-15 Alkyl Benzoate | 21,8 |
| Dermofeel BGC | Butylene Glycol Caprylate/Caprate | 21,5 |
| Miglyol 812 | Caprylic/Capric Triglyceride | 21,3 |
| Trivent OCG | Tricaprylin | 20,2 |
| Dermol 866 | PEG " Diethylhexanoate/ Diisononanoate/ Ethylhexyl Isononanoate | 20,1 |

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

| **Handelsname** | **INCI-Name** | **Polarität [m/Nm]** |
|---|---|---|
| Ecolane 130 | Cycloparaffin | 49,1 |
| Nexbase 2006 FG | Polydecene | 46.7 |
| Polysynlane | Hydrogenated Polyisobutene | 44.7 |
| Wacker Silikonöl AK 50 | Polydimethylsiloxan | 46,5 |
| Solvent ICH | Isohexadecane | 43.8 |
| Pionier 2076 | Mineral Oil | 43.7 |
| Pionier 6301 | Mineral Oil | 43.7 |
| Wacker Silikonöl AK 35 | Polydimethylsiloxan | 42,4 |
| Isoeikosan | Isoeikosan | 41.9 |
| Wacker Silikonöl AK 20 | Polydimethylsiloxan | 40,9 |
| Isofol 1212 Carbonat | | 40,3 |
| Softcutol O | Ethoxydiglycol Oleate | 40,5 |
| Lipodermanol OL | Decyl Olivate | 40,3 |
| Cetiol S | Dioctylcyclohexane | 39,0 |
| Pionier 2071 | Mineral Oil | 38.3 |
| Hydrobrite 1000 PO | Paraffinum Liquidum | 37,6 |
| Tegosoft HP | Isocetyl Palmitate | 36,2 |
| Isofol Ester 1693 | | 33,5 |
| Isofol Ester 1260 | | 33,0 |
| Dow Corning Fluid 245 | Cyclopentasiloxan | 32,3 |
| Prisorine 2036 | Octyl Isostearate | 31.6 |
| Cetiol CC | Dicaprylyl Carbonate | 31,7 |
| Dermol 99 | Trimethylhexyl Isononanoate | 31,1 |
| Dermol 89 | 2- Ethylhexyl Isononanoate | 31,0 |
| Cetiol OE | Dicaprylyl Ether | 30,9 |
| Dihexylcarbonat | Dihexyl Carbonate | 30,9 |
| Silkflo 366 NF | Polydecene | 30,1 |
| Estol 1540 EHC | Octyl Cocoate | 30.0 |

Es ist jedoch auch vorteilhaft, Gemische aus höher- und niederpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen. Auch beliebige Abmischungen solcher Öl- und Wachs-komponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

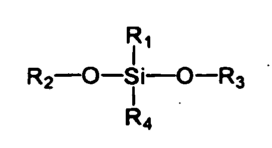

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 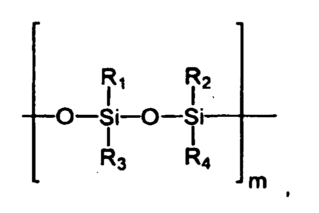 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 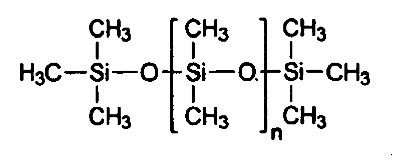 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

| **Handelsname** | **INCI-Name** | **Polarität [mN/m]** |
|---|---|---|
| Wacker Silikonöl AK 100 | Polydimethylsiloxan | 26,9 |
| Wacker Silikonöl AK 50 | Polydimethylsiloxan | 46,5 |
| Wacker Silikonöl AK 35 | Polydimethylsiloxan | 42,4 |
| Wacker Silikonöl AK 20 | Polydimethylsiloxan | 40,9 |
| Dow Corning Fluid 245 | Cyclopentasiloxan | 32,3 |
| Dow Corning Fluid 345 | Cyclomethicone | 28,5 |

Erfindungsgemäß vorteilhaft einzusetzende cyclische Silicone werden im allgemeinen durch Strukturelemente charakterisiert, wie folgt 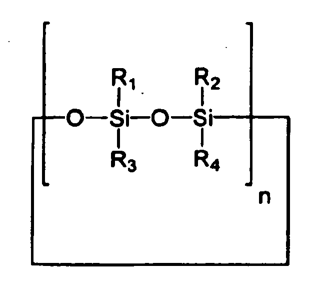 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxanpolyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Die wäßrige Phase der erfindungsgemäßen Formulierungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Xanthangummi und/oder Hydroxypropylmethylcellulose, jeweils einzeln oder in Kombination.

Es ist auch vorteilhaft im Sinne der vorliegenden Erfindung, kosmetische und dermatologische Zubereitungen zu erstellen, deren hauptsächlicher Zweck zwar nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und - gewünschtenfalls - Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Nachfolgende Beispiele erläutern die Erfindung. Die Anteilsangaben beziehen sich als Gewichtsprozent jeweils auf die Gesamtmasse der Zubereitung. Die erfindungsgemäßen Beispielzubereitungen weisen einen leichten Grünton auf und gibt auf gereizter, geröteter Haut aufgetragen der Haut ein natürliches Aussehen. Der Farbabstufung der Zubereitungen kann im beanspruchten Bereich je nach Umgebungsparametern, Hautfarbe, Rötungsgrad individuell angepasst werden.

### Beispiele

**W/O-Emulsionen**

| **Beispiel** | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | -- |
| Glycyrrhiza Inflata | 0,025 | 0,035 | --- | --- | 0,25 |
| Hammamelis | 1,0 | 2,0 | --- | 5,0 | --- |
| Wasser | ad 100 | ad 100 | ad 100 | ad 100 | ad 100 |

| **Beispiel** | **6** | **7** | **8** | **9** | **10** |
|---|---|---|---|---|---|
| PEG-30 Dipolyhydroxystearat | --- | 0,5 | 0,25 | --- | 3,0 |
| Lanolin Alcohol | 1,0 | 1,5 | 1,75 | 3,0 | --- |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0, 75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | --- |
| Glycyrrhiza Inflata | 0,025 | 0,035 | --- | --- | 0,25 |
| Hammamelis | 1,0 | 2,0 | --- | 5,0 | --- |
| Kaliumsorbat | 0,3 | 0,4 | 0,25 | 0,15 | --- |
| Wasser | ad 100 | ad 100 | ad 100 | ad 100 | ad 100 |

**W/S-Emulsion**

| **Beispiel** | **11** | **12** | **13** | **14** | **15** |
|---|---|---|---|---|---|
| Cetyl PEG/PPG-10/1 Dimethicone | 1,0 | --- | --- | 3,0 | 5,0 |
| Cylomethicon + PEG/PPG-18/18 Dimethicon (90:10) | 10,0 | 12,5 | 25 | --- | --- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Weisspigment (CI 77891) | 2,5 | 3.0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1.0 | 2,0 | 3,0 | --- | --- |
| Glycyrrhiza Inflata | 0,025 | 0,035 | --- | --- | 0,25 |
| Hammamelis | 1,0 | 2,0 | --- | 5,0 | --- |
| Cetyldimethicon | 0,5 | --- | 0,7 | --- | --- |
| Benzylalkohol | --- | --- | 0,05 | --- | 0,1 |
| Wasser | ad 100 | ad 100 | ad 100 | ad 100 | ad 100 |

| **Beispiel** | **16** | **17** | **18** | **19** | **20** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q.s, | q,s, | q,s, |
| Stearyldimethicon | 0,5 | --- | 0.7 | --- | --- |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | --- |
| Glycyrrhiza Inflata | 0,025 | 0,035 | --- | --- | 0,25 |
| Hammamelis | 1,0 | 2,0 | --- | 5,0 | --- |
| Dehydracetsäure | --- | --- | 0,05 | --- | 0,1 |
| modifizierte Stärke | --- | 2,5 | --- | 0,15 | --- |
| Wasser | ad 100 | ad 100 | ad 100 | ad 100 | ad 100 |

**W/O-Emulsionen**

| **Beispiel** | **21** | **22** | **23** | **24** | **25** |
|---|---|---|---|---|---|
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | --- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0.1 | 0,05 | 0,15 | 0,5 | 1,0 |
| hydriertes Rizinusöl | 0,5 | 0,75 | 1,0 | 2,5 | 5,0 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Citronensäure | 0,2 | 0,1 | --- | --- | --- |
| Parfum | q,s, | q,s, | q,s, | q,s, | q,s, |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1.0 | 2,0 | 3,0 | --- | --- |
| Glycyrrhiza Inflata | 0,025 | 0,035 | --- | --- | 0,25 |
| Hammamelis | 1,0 | 2,0 | --- | 5,0 | --- |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | --- | --- | 0,05 | --- | 0,1 |
| Wasser | ad 100 | ad 100 | ad 100 | ad 100 | ad 100 |

| **Beispiel** | **26** | **27** | **28** | **29** | **30** |
|---|---|---|---|---|---|
| Polyglyceryl-2-dipolyhydroxystearat | 3,0 | --- | 0,25 | --- | 3,0 |
| Polyglyceryl-3 diisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5.0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Magnesiumsulfat | 0,5 | 0,6 | 0,5 | 0,7 | 1,0 |
| Glycerin | 3,0 | 5,0 | 10,0 | 15,0 | 1,5 |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | --- |
| Glycyrrhiza Inflata | 0,025 | 0,035 | --- | --- | 0,25 |
| Hammamelis | 1,0 | 2,0 | --- | 5,0 | --- |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

**Silikon in Wasser-Emulsion**

| **Beispiel** | **31** | **32** | **33** | **34** | **35** |
|---|---|---|---|---|---|
| Dimethiconcopolyol, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Xanthan Gum | --- | 0,1 | --- | 0,25 | 1.0 |
| Parfum | q,s, | q,s, | q,s, | q,s, | q,s, |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | --- |
| Glycyrrhiza Inflata | 0,025 | 0,035 | --- | --- | 0,25 |
| Hammamelis | 1,0 | 2,0 | --- | 5,0 | --- |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Wasser | ad 100 | ad 100 | ad 100 | ad 100 | ad 100 |

**O/W-Emulsion**

| **Beispiel** | **36** | **37** | **38** | **39** | **40** |
|---|---|---|---|---|---|
| Glycerylstearat | 1,0 | --- | --- | 0,5 | 0,25 |
| Polyethylenglycol(40)stearat | 10,0 | --- | 5 | --- | --- |
| Triglycerinmethylglucosedistearat | --- | 5,5 | --- | --- | 2,5 |
| Sorbitanstearat | --- | 1,5 | 3 | --- | --- |
| Cyclomethicon | 2,5 | 15 | 8,0 | 5,0 | 7,5 |
| Dimethicon | 5,0 | 3,0 | 5,0 | 2,0 | 5,0 |
| Behenylalkohol | 1 | --- | 2 | 1 | --- |
| Stearylalkohol | --- | 1 | --- | 1 | ---- |
| Cetylstearylalkohol | zu | zu | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0.1 |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | --- |
| Glycyrrhiza Inflata | 0,025 | 0,035 | --- | --- | 0,25 |
| Hammamelis | 1,0 | 2,0 | --- | 5,0 | --- |
| Glycerin | 5 | 10 | 3 | 15 | 7,5 |
| modifizierte Stärke | --- | 2,5 | --- | 0,15 | --- |
| Wasser | ad 100 | ad 100 | ad 100 | ad 100 | ad 100 |

**O/W-Emulsion**

| **Beispiel** | **41** | **42** | **43** | **44** | **45** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Glycerin | 5 | 10 | 15 | 3 | 7.5 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | --- |
| Glycyrrhiza Inflata | 0,025 | 0,035 | --- | --- | 0,25 |
| Hammamelis | 1.0 | 2,0 | --- | 5,0 | --- |
| modifizierte Stärke | 0,5 | --- | --- | 0,15 | -- |
| Wasser | ad 100 | ad 100 | ad 100 | ad 100 | ad 100 |

**O/W-Emulsion**

| **Beispiel** | **46** | **47** | **48** | **49** | **50** |
|---|---|---|---|---|---|
| Glycerylsteratcitrat | 1,0 | 0,5 | 0,1 | 0,5 | 0,3 |
| Polyethylenglycol(20)cetearylether | 10,0 | 1,0 | 5 | -- | -- |
| Triglycerinmethylglucosedistearat | --- | --- | --- | --- | 2,5 |
| Dimethicon | 0,5 | 3,0 | 0,75 | 1,5 | 0,2 |
| Dicaprylylcarbonat | 3 | 5 | 10 | 15 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Iodopropynylbutylcarbamat | --- | --- | 0,05 | --- | 0,1 |
| Phenoxyethanol | --- | 0,5 | --- | 0,15 | --- |
| Sorbitol | 10 | --- | --- | 5 | --- |
| Butylenglykol | --- | --- | --- | 5 | 10 |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | 1,0 | 2,0 | 3,0 | --- | --- |
| Glycyrrhiza Inflata | 0,025 | 0,035 | --- | --- | 0.25 |
| Hammamelis | 1,0 | 2,0 | --- | 5,0 | --- |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

| **Beispiel** | **51** | **52** | **53** | **54** | **55** |
|---|---|---|---|---|---|
| Sorbitanstearat | 0,86 | --- | --- | 2,7 | 2,6 |
| Titandioxid | 1,5 | 4,0 | 3,75 | 3,1 | 5,5 |
| Sheabutter | 1,5 | 2,5 | 3,75 | --- | 3,0 |
| Triglycerinmethylglucosedistearat | 2,14 | 3,0 | 3,0 | 0,6 | 1,2 |
| Squalan | 2,0 | --- | --- | 4,0 | 3,5 |
| Dimethicon | 4,0 | 3,0 | 5,0 | 3,0 | 2,75 |
| Lauroyllysin | 2,0 | 3,0 | 1,5 | 1,25 | --- |
| Ethylhexylmethoxyzimtsäureester | 7,5 | --- | --- | 1,3 | 1,3 |
| C12-15 Alkylbenzoat | 2,0 | 2,5 | 3,75 | 3,0 | 4,5 |
| Phenoxyethanol | 0,5 | 0,4 | 0,4 | 0,75 | 0,5 |
| Methylparaben | 0,3 | 0,2 | 0,2 | 0,2 | 0,2 |
| Glycerin | 10,0 | 10,0 | 10,0 | 10,0 | 12,5 |
| Propylparaben | 0,1 | 0,1 | 0,1 | 0,1 | 0,1 |
| Xanthangummi | 0,2 | 0,1 | 0,1 | 0,2 | 0,1 |
| Weisspigment (CI 77891) | 2,5 | 3,0 | 2,25 | 3,5 | 5,0 |
| Grünpigment (CI 77288) | 0,27 | 0,42 | 0,54 | 0,35 | 0,75 |
| Grünpigment (CI 77288) | 0,088 | 0,1 | 0,16 | 0,095 | 0,25 |
| Blaupigment (CI 77007) | 0,03 | 0,04 | 0,05 | 0,03 | 0,08 |
| Panthenol | --- | 2,0 | 3,0 | --- | --- |
| Glycyrrhiza Inflata | 0,025 | 0,035 | --- | --- | 0,25 |
| Hammamelis | --- | 2,0 | --- | 5,0 | --- |
| Parfum | q,s, | q,s, | q,s, | q,s, | q,s, |
| Magnesiumaluminiumsilikat | 0,3 | --- | --- | --- | 0,5 |
| Butylenglykol | 0,5 | 0,018 | 0,018 | --- | 0,018 |
| Wasser | ad 100 | ad 100 | ad 100 | ad 100 | ad 100 |

**Emulsionen für Concealer-Applikation**

| **Beispiel** nicht erfindungsgemäß | **56** | **57** | **58** | **59** | **60** |
|---|---|---|---|---|---|
| Triglycerindiisostearat | 5,12 | 4,25 | 3,75 | 6,22 | 5,26 |
| Cyclopentasiloxan | 14,8 | 10,8 | 8,5 | 16,7 | 12,3 |
| Propylenglykol Dicaprylat/Dicaprat | 6,23 | 10;0 | 8,0 | 5,0 | 5,96 |
| Methylmethacrylacrosspolymer | 4,15 | 3,18 | 5,0 | 4,35 | 2,5 |
| Glyceryloleat | 2,5 | 1,5 | 2,5 | 3,5 | 2,75 |
| Vaseline | 2,23 | 3,23 | 1,23 | 1,95 | 2,45 |
| Candelillawachs | 1,11 | 1,0 | 0,75 | 2,2 | 0,25 |
| Carnaubawachs | 1,11 | 0,3 | 0,6 | 1,0 | 0,25 |
| Acrylat/Octylacrylamid Coipolymer | 1,0 | 1,5 | 0,75 | 1,25 | 1,0 |
| Dimethiconcrosspolymer | 0,67 | 0,75 | 1,0 | 0,5 | 0,88 |
| Panthenol | 0,5 | 0,6 | 0,5 | 0,7 | 1,0 |
| Glycerin | 8,5 | 5,0 | 10,0 | 15,0 | 1,5 |
| Butylenglykol | 0,3 | 0,1 | 0,2 | 0,3 | 1,0 |
| EDTA, Dinatrium-Salz | 0,2 | 0.05 | 0.4 | 0.3 | 0,18 |
| Parfum | --- | q,s, | --- | q,s, | --- |
| PEG/PPG-18/18 Dimethicon | 0,11 | 0,05 | 0,25 | 0,12 | 0,13 |
| Weisspigment (CI 77891) | 8,0 | 10,0 | 6,0 | 8,3 | 5,0 |
| Grünpigment (CI 77288) | 0,81 | 0,42 | 0,54 | 0,35 | 0,75 |
| Eisenoxid (CI 77492) | 0,152 | 0,1 | 0,16 | 0,095 | 0,25 |
| Mica | 1,67 | 1,87 | 1,47 | 1,65 | --- |
| Glycyrrhiza Inflata | 0,025 | 0,035 | 0,025 | 0,01 | 0,25 |
| Diazolidinyl Urea | 0,2 | 0,2 | 0,2 | 0,18 | 0,2 |
| Iodopropynylbutylcarbamat | 0,016 | 0,018 | 0,016 | 0,018 | 0,018 |
| Phenoxyethanol | 0,15 | 0,25 | 0,15 | 0,2 | 0,3 |
| Hamamelis | --- | 2,0 | --- | 5,0 | --- |
| Wasser | ad 100 | ad 100 | ad 100 | ad 100 | ad 100 |

## Patentansprüche

1. Kosmetische oder dermatologische Zubereitungen enthaltend 0,01 Gew.% bis 5 Gew.% mindestens eines rotlichtfilternden Farbstoffes, 0,0001 Gew.% - 10 Gew.% mindestens eines antiinflammatorischen Wirkstoffes, gewählt wird aus der Gruppe Hammamelis, Glycyrhizza inflata, Panthenol und/oder Kamillenextrakt oder Licochalcon A, ein oder mehrere Weißpigmente und ein oder mehrere Blaupigmente im Verhältnis 1:1 bis 1:100 im Bezug auf das eingesetzte Grünpigment.

2. Zubereitung nach Anspruch 1, **dadurch gekennzeichnet, dass** der Wirkstoff mit einem Anteil von 0,01 bis 7 Gew.%, bevorzugt 0,05 bis 5 Gew.-%, insbesondere 0,1 bis 2 Gew.-% enthalten ist.

3. Verwendung von 0,01 Gew.% bis 5 Gew.% mindestens eines rotlichtfilternden Farbstoffes, 0,0001 Gew.% bis 10 Gew.% mindestens eines antiinflammatorischen Wirkstoffes, gewählt wird aus der Gruppe Hammamelis, Glycyrrhiza inflata, Panthenol und/oder Kamillenextrakt oder Licochalcon A, ein oder mehrere Weißpigmente und ein oder mehrere Blaupigmente im Verhältnis 1:1 bis 1:100 im Bezug auf das eingesetzte Grünpigment zur Herstellung kosmetischer oder dermatologischer Zubereitungen zur Pflege licht- und/oder rasurstrapazierter Haut und/oder zur Linderung oder der Nachreaktionen der Haut auf die Einwirkung von Sonnen- und/oder UV-Strahlung und/oder der Rasur sowie zur Behandlung und Pflege krankheitsbedingt, gereizt oder entzündlich rotgefarbter Haut, insbesondere Rosacea.

4. Verwendung von 0,01 Gew.% bis 5 Gew.% mindestens eines rotlichtfilternden Farbstoffes, 0,0001 Gew.% bis 10 Gew.% mindestens eines antiinflammatorischen Wirkstoffes, gewählt wird aus der Gruppe Hammamelis, Glycyrhizza inflata, Panthenol und/oder Kamillenextrakt oder Licochalcon A, ein oder mehrere Weißpigmente und ein oder mehrere Blaupigmente im Verhältnis 1:1 bis 1:100 im Bezug auf das eingesetzte Grünpigment zur Herstellung kosmetischer oder dermatologischer Zubereitungen zur visuelle Kaschierung von Rosacea und/oder geröteter Haut, insbesondere durch Sonnebestrahlung verursacht, und gleichzeitig zur langfristigen antiinflammatorischen Pflege der Haut.

## Claims

1. Cosmetic or dermatological preparations comprising 0.01% by weight to 5% by weight of at least one red light-filtering dye, 0.0001% by weight - 10% by weight of at least one antiinflammatory active ingredient, is chosen from the group consisting of hamamelis, Glycyrhizza inflata, panthenol and/or camomile extract or licochalcone A, one or more white pigments and one or more blue pigments in the ratio 1:1 to 1:100, based on the green pigment used.

2. Preparation according to Claim 1, **characterized in that** the active ingredient is present in an amount of from 0.01 to 7% by weight, preferably 0.05 to 5% by weight, in particular 0.1 to 2% by weight.

3. Use of 0.01% by weight to 5% by weight of at least one red light-filtering dye, 0.0001% by weight to 10% by weight of at least one antiinflammatory active ingredient, is chosen from the group consisting of hamamelis, Glycyrhizza inflata, panthenol and/or camomile extract or licochalcone A, one or more white pigments and one or more blue pigments in the ratio 1:1 to 1:100, based on the green pigment used, for the preparation of cosmetic or dermatological preparations for the care of photodamaged and/or shaving-stressed skin and/or for the alleviation or of the after-reactions of the skin to the effect of solar and/or UV radiation and/or shaving, and for the treatment and care of reddened skin caused by disease, irritation or inflammation, in particular rosacea.

4. Use of 0.01% by weight to 5% by weight of at least one red light-filtering dye, 0.0001% by weight to 10% by weight of at least one antiinflammatory active ingredient, is chosen from the group consisting of hamamelis, Glycyrhizza inflata, panthenol and/or camomile extract or licochalcone A, one or more white pigments and one or more blue pigments in the ratio 1:1 to 1:100, based on the green pigment used, for the preparation of cosmetic or dermatological preparations for the visual concealment of rosacea and/or reddened skin, in particular that caused by solar irradiation, and at the same time for the long-term antiinflammatory care of the skin.

## Revendications

1. Préparations cosmétiques ou dermatologiques contenant de 0,01 % en poids à 5 % en poids d'au moins un colorant filtrant la lumière rouge, de 0,0001 % en poids à 10 % en poids d'au moins une substance active anti-inflammatoire, est choisie dans le groupe constitué par l'hamamélis, le réglisse Glycyrhizza inflata, le panthénol et/ou l'extrait de camomille ou la Licochalcone A, un ou plusieurs pigments blancs et un ou plusieurs pigments bleus en un rapport de 1:1 à 1:100 par rapport au pigment vert utilisé.

2. Préparation selon la revendication 1, **caractérisée en ce que** la substance active est contenue en une proportion de 0,01 à 7 % en poids, de préférence de 0,05 à 5 % en poids, en particulier de 0,1 à 2 % en poids.

3. Utilisation de 0,01 % en poids à 5 % en poids d'au moins un colorant filtrant la lumière rouge, de 0,0001 % en poids à 10 % en poids d'au moins une substance active anti-inflammatoire, est choisie dans le groupe constitué par l'hamamélis, le réglisse Glycyrhizza inflata, le panthénol et/ou l'extrait de camomille ou la Licochalcone A, d'un ou plusieurs pigments blancs et d'un ou plusieurs pigments bleus en un rapport de 1:1 à 1:100 par rapport au pigment vert utilisé, pour la fabrication de préparations cosmétiques ou dermatologiques pour le soin de la peau abîmée par la lumière et/ou le rasage et/ou pour l'apaisement ou des réactions de la peau faisant suite à l'action du rayonnement solaire et/ou UV et/ou du rasage, ainsi que pour le traitement et le soin de la peau rougie à cause d'une maladie, d'une irritation ou d'une inflammation, en particulier de l'acné rosacée.

4. Utilisation de 0,01 % en poids à 5 % en poids d'au moins un colorant filtrant la lumière rouge, de 0,0001 % en poids à 10 % en poids d'au moins une substance active anti-inflammatoire, est choisie dans le groupe constitué par l'hamamélis, le réglisse Glycyrhizza inflata, le panthénol et/ou l'extrait de camomille ou la Licochalcone A, d'un ou plusieurs pigments blancs et d'un ou plusieurs pigments bleus en un rapport de 1:1 à 1:100 par rapport au pigment vert utilisé, pour la fabrication de préparations cosmétiques ou dermatologiques destinées au masquage visuel de l'acné rosacée et/ou de la peau rougie, en particulier causée par le rayonnement solaire, et en même temps pour le soin anti-inflammatoire à long cours de la peau.
